# EUROPEAN PATENT APPLICATION

(11) **EP 4 520 382 A1**
(43) Date of publication of application: **12.03.2025**
(21) Application number: 23816138.4
(22) Date of filing: 01.06.2023
(51) Int. Cl.: A61M 31/00, A61M 37/00, A61F 2/16, A61B 17/00, C12M 1/00

(54) **TRANSFER INSTRUMENT**

(30) Priority: 01.06.2022 JP 2022089711
(71) Applicant: Terumo Kabushiki Kaisha, Tokyo 151-0072 (JP)
(72) Inventor: HAYAKAWA, Koichi, Ashigarakami-gun, Kanagawa 259-0151 (JP)
(74) Representative: Prüfer & Partner mbB Patentanwälte · Rechtsanwälte
(86) International application number: PCT/JP2023/020397
(87) International publication number: WO 2023/234381

(57) **Abstract**

A transfer instrument (10) transfers a medical sheet (300) to a recipient site (402) of a heart (400). A transfer instrument (10) includes a first carrier member (18) including a first shaft (24) and a support portion (26), the support portion (26) including a support surface (46) on which the medical sheet (300) is placed, and a second carrier member (20) including a second shaft (54) and a pressing portion (50). The second carrier member (20) is movable relative to the first carrier member (18) so as to allow a pressing surface (64) of the pressing portion (50) to press, in a distal end direction, an outer edge surface (302) of the medical sheet (300) placed on the support surface (46).

## Description

### Technical Field

The present invention relates to a transfer instrument.

### Background Art

JP 2009-511 A discloses a transfer instrument for transferring a medical sheet (cell sheet) for use in, for example, organ transplantation to a treatment site of a living body. The transfer instrument includes a shaft and a support portion provided at a distal portion of the shaft. The support portion includes a support surface on which the medical sheet is placed. The transfer instrument is slid with an upper surface of the medical sheet placed on the support surface pressed with forceps or the like to transfer the medical sheet from the support portion to the treatment site.

### Summary of Invention

The above-described related art requires that, when transferring the medical sheet from the support portion to the treatment site of the living body, the transfer instrument be manipulated with the upper surface of the medical sheet pressed with forceps or the like, so that there is possibility that the medical sheet cannot be efficiently transferred to the treatment site.

It is therefore an object of the present invention to solve the above-described problem.

(1) An aspect of the present invention is a transfer instrument that transfers a medical sheet to a treatment site of a living body, the transfer instrument including: a first carrier member including a first shaft and a support portion, the support portion being provided at a distal portion of the first shaft and including a support surface on which the medical sheet is placed; and a second carrier member including a second shaft and a pressing portion, the second shaft extending along an axial direction of the first shaft, the pressing portion being provided at a distal portion of the second shaft, in which the second carrier member is movable relative to the first carrier member so as to allow a pressing surface of the pressing portion to press, in a distal end direction, an outer edge surface of the medical sheet placed on the support surface.
   According to the present invention, it is possible to slide, by causing the pressing surface of the pressing portion to press, in the distal end direction, the outer edge surface of the medical sheet placed on the support surface of the first carrier member, the medical sheet from the support surface to the treatment site of the living body. It is therefore possible to efficiently transfer the medical sheet to the treatment site.
(2) In the transfer instrument according to the above (1), the first shaft may be formed in a tubular shape, and the second shaft may be inserted into a lumen of the first shaft so as to be movable along the axial direction of the first shaft.
(3) In the transfer instrument according to the above (1) or (2), the pressing portion may slide on the support surface when the outer edge surface of the medical sheet is pressed in the distal end direction by the pressing surface.
(4) In the transfer instrument according to any one of the above (1) to (3), the second carrier member may include a liquid supply flow path through which a liquid is supplied to the medical sheet placed on the support surface.
(5) In the transfer instrument according to any one of the above (1) to (4), the pressing surface may extend upward with an inclination in the distal end direction.
(6) In the transfer instrument according to any one of the above (1) to (5), the pressing portion may be formed to be wider in the distal end direction when viewed from above.
(7) In the transfer instrument according to any one of the above (1) to (4), the pressing surface may be curved in an arc shape in a proximal end direction when viewed from above.
(8) The transfer instrument according to the above (2) may further include an outer cylinder through which the first shaft is inserted, in which the support portion may be flexible and formed in a sheet shape, the support portion may be formed to be wider than an inner diameter of the outer cylinder, the support portion and the medical sheet may be retracted into the outer cylinder with the support portion and the medical sheet undergoing curved deformation when the first shaft and the second shaft are moved in a proximal end direction relative to the outer cylinder, and the support portion and the medical sheet retracted in the outer cylinder may be unfolded when the first shaft and the second shaft are moved in the distal end direction relative to the outer cylinder to be exposed from the outer cylinder.
(9) In the transfer instrument according to the above (8), a maximum width of the pressing portion may be smaller than a width of the support portion and larger than an outer diameter of the second shaft.
(10) In the transfer instrument according to the above (8) or (9), the pressing portion may be flexible, and a width of the pressing surface may be larger than the inner diameter of the outer cylinder.
(11) In the transfer instrument according to any one of the above (8) to (10), the support portion may include a pair of protrusions protruding upward from both sides of the support surface in a width direction.
(12) In the transfer instrument according to the above (11), the pressing portion may be provided with a retaining slit into which the pair of protrusions are inserted in a retracted state where the support portion and the medical sheet are retracted in the outer cylinder.
(13) The transfer instrument according to any one of the above (1) to (12) may further include an endoscope that captures an image of the support portion, the pressing portion, and the medical sheet.

### Brief Description of Drawings

Fig. 1 is a perspective view of a transfer instrument according to an embodiment of the present invention.
Fig. 2 is an exploded perspective view of the transfer instrument illustrated in Fig. 1.
Fig. 3 is a plan view of a distal portion of the transfer instrument illustrated in Fig. 1.
Fig. 4 is a longitudinal cross-sectional view taken along line IV-IV in Fig. 3.
Fig. 5 is a transverse cross-sectional view taken along line V-V in Fig. 3.
Fig. 6 is a flowchart illustrating a procedure of a transfer method for transferring a medical sheet using the transfer instrument illustrated in Fig. 1.
Fig. 7 is a first explanatory diagram of a sheet placing process.
Fig. 8 is a second explanatory diagram of the sheet placing process.
Fig. 9 is an explanatory diagram of a retracting process.
Fig. 10 is a transverse cross-sectional view taken along line X-X in Fig. 9.
Fig. 11 is a transverse cross-sectional view taken along line XI-XI in Fig. 9.
Fig. 12 is an explanatory diagram of a positioning process.
Fig. 13 is an explanatory diagram of an unfolding process.
Fig. 14 is a first explanatory diagram of a moving process.
Fig. 15 is a second explanatory diagram of the moving process.
Fig. 16A is a partially omitted schematic view of a transfer instrument including an instrument body according to a first modification. Fig. 16B is a transverse cross-sectional view taken along line XVIB-XVIB in Fig. 16A.
Fig. 17 is an explanatory diagram of a state where a second carrier member illustrated in Fig. 16A is moved in a distal end direction.
Fig. 18 is a partially omitted perspective view of a second carrier member including a pressing portion according to a first configuration example.
Fig. 19 is a partially omitted perspective view of a second carrier member including a pressing portion according to a second configuration example.
Fig. 20 is a partially omitted perspective view of a second carrier member including a pressing portion according to a third configuration example.
Fig. 21 is a perspective view of a transfer instrument including an instrument body according to a second modification.
Fig. 22 is a transverse cross-sectional view of the transfer instrument illustrated in Fig. 21, illustrating a state where a support portion is retracted in an outer cylinder.

### Description of Embodiments

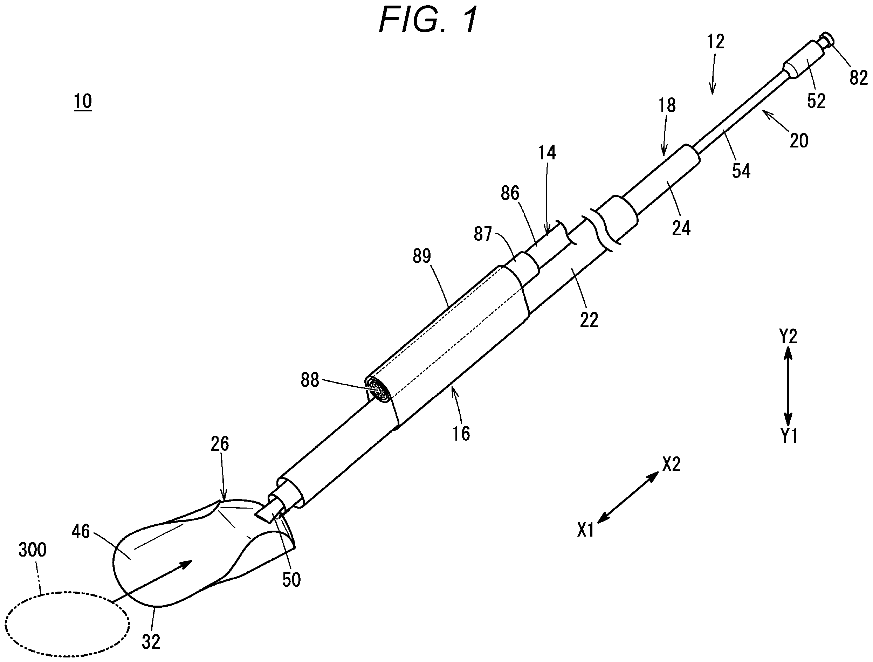

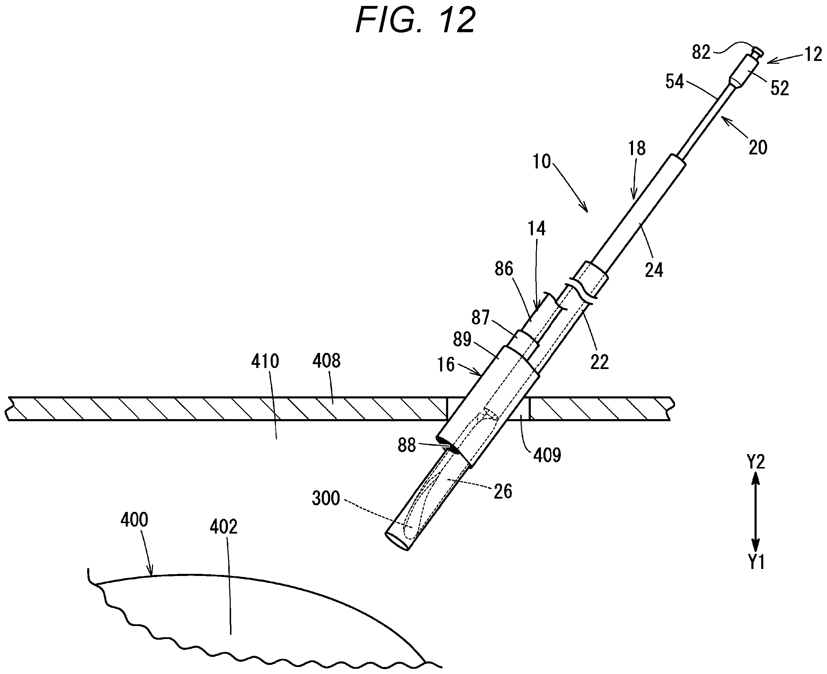

As illustrated in Fig. 1, a transfer instrument 10 according to an embodiment of the present invention is a medical instrument for transferring a medical sheet 300 to a treatment site of a living body. The transfer instrument 10 is used for, for example, the treatment of severe heart failure caused by ischemic heart disease. In this case, the medical sheet 300 is transplanted to a recipient site 402 of a heart 400 (the treatment site of the living body) (see Figs. 12 to 15). The transfer instrument 10 is capable of attaching a plurality of the medical sheets 300 to the recipient site 402.

Examples of such a medical sheet 300 include pharmaceutical products or regenerative medicine products for medical use, a medical instrument, and the like. The medical sheet 300 is formed in a sheet shape such as a film shape or a membrane shape (high viscosity object). Fibrin or the like may be applied to the medical sheet 300 for reinforcement. Examples of the regenerative medicine products including cells include a cell sheet (sheet-shaped cell culture), a spheroid (multicellular spheroid ), and the like. It is possible to form the cell sheet by culturing autologous cells or allogenic cells. The cells constituting the cell sheet include, for example, somatic stem cells (adult stem cells), mesenchymal stem cells, or iPS cells (induced pluripotent stem cells)-derived cardiomyocytes. Examples of the somatic stem cells preferably include skeletal myoblast cells (myoblast cells).

The medical sheet 300 may contain a tissue adhesive, a local anesthetic, or the like. The medical sheet 300 has a thickness of, for example, about 100 µm, and has a diameter of, for example, about 60 mm. Note that the thickness and the diameter (size) of the medical sheet 300 can be set as desired.

The medical sheet 300 may be a sheet to be transplanted to an organ (for example, lung, liver, pancreas, kidney, small intestine, esophagus, or the like) other than the heart 400. Further, the medical sheet 300 may be, for example, an anti-adhesion sheet as long as the sheet is for medical use.

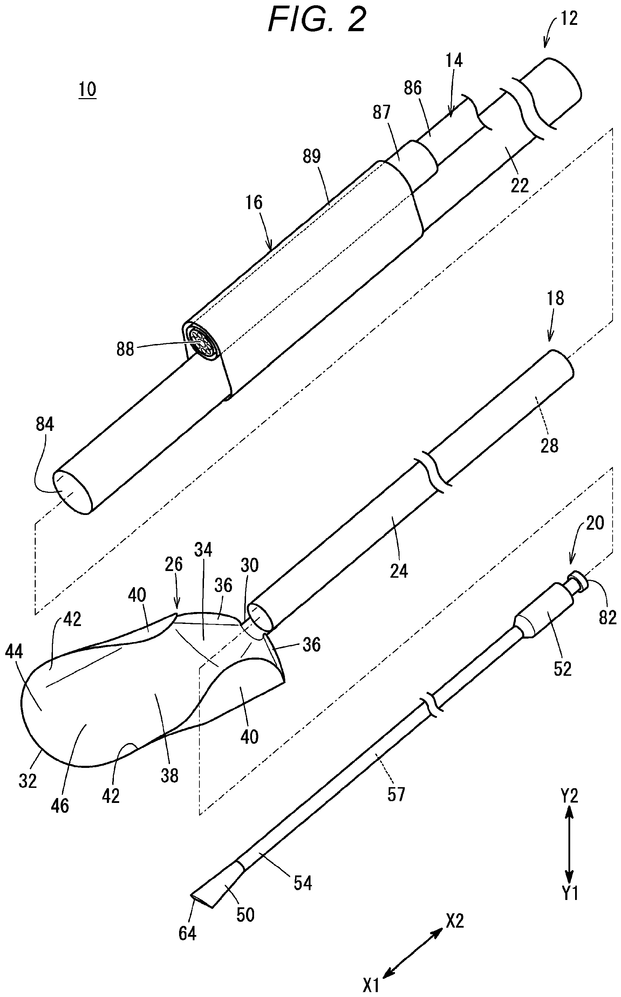

As illustrated in Figs. 1 and 2, the transfer instrument 10 includes an instrument body 12, an endoscope 14, and a fixing member 16. The instrument body 12 includes a first carrier member 18, a second carrier member 20, and an outer cylinder 22.

In Fig. 2, the first carrier member 18 includes a first shaft 24 and a support portion 26. The first shaft 24 is a circular tube member having a first lumen 28. The first lumen 28 opens at a distal end (end in a direction of arrow X1) of the first shaft 24 and opens at a proximal end (end in a direction of arrow X2) of the first shaft 24.

The first shaft 24 includes, for example, a resin material. Examples of the constituent material of the first shaft 24 include, but not particularly limited to, polyethylene, polypropylene, fluororesin, polyethylene terephthalate, polymethyl methacrylate, polyamide resin, polystyrene, polycarbonate, polyimide, polyetherimide, polyetheretherketone, polyvinyl chloride, and ABS resin. The first shaft 24 may include a metal material.

The first shaft 24 may be flexible. The first shaft 24 may include a flexible tube portion capable of retaining a bent shape. In this case, the first shaft 24 can be bent into an any desired shape in a body cavity and can retaining the bent shape. The flexible tube portion is formed by, for example, forming a tube wall portion in a pleated shape. Note that the flexible tube portion may include a material capable of retaining a bent shape. Specifically, the flexible tube portion can be formed, for example, by inserting a linear metal member capable of retaining a bent shape into the lumen of the first shaft 24 including resin. The flexible tube portion forms only a part of the first shaft 24 in a longitudinal direction. Note that the flexible tube portion may form the entire first shaft 24.

As illustrated in Figs. 2 to 4, the support portion 26 is attached to a distal portion of the first shaft 24. The support portion 26 includes, for example, a resin material. The constituent material of the support portion 26 preferably has transparency, and examples of the constituent material include, but not particularly limited to, polycarbonate, polyamide, polystyrene, polypropylene, polyacetal resin, polyimide, polyetheretherketone, polyethylene terephthalate, and fluororesin.

The support portion 26 is flexible. A resin sheet member (film member) is bent into a predetermined form to form the support portion 26. Alternatively, the sheet member is shaped into a predetermined form by a sheet forming die to form the support portion 26. It is preferable that the sheet member have, but not particularly limited to, a thickness of, for example, 100 µm or more and 200 µm or less. The support portion 26 includes a joint 30 and a support body 32.

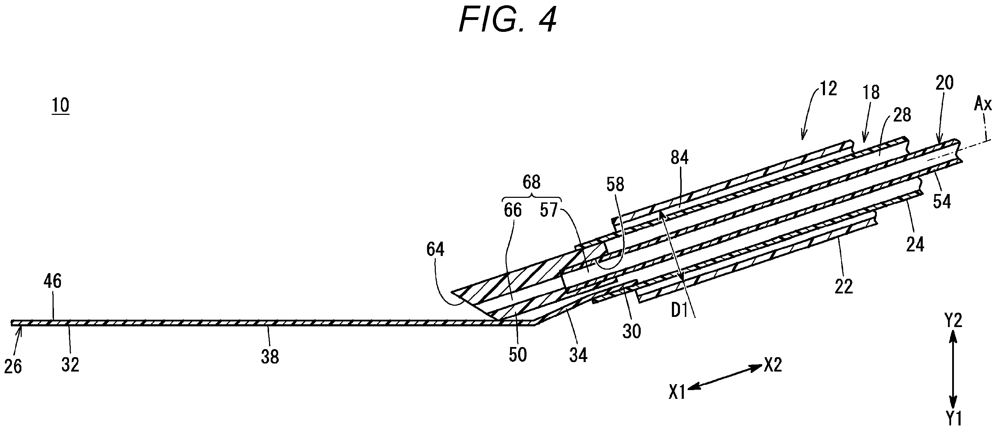

In Fig. 4, the joint 30 is bonded to an inner peripheral surface of the distal portion of the first shaft 24 with an adhesive. Examples of the adhesive include, but not particularly limited to, a UV adhesive and an instant adhesive (for example, cyanoacrylate-based instant adhesive). The joint 30 may be thermally fused to the inner peripheral surface of the first shaft 24. In a case where the joint 30 is joined to the inner peripheral surface of the distal portion of the first shaft 24, the joint 30 does not form a step on an outer peripheral surface of the first shaft 24, so that the first shaft 24 can be smoothly inserted into the outer cylinder 22. The joint 30 may be joined to a lower surface (an outer surface facing the direction of arrow Y1) of the distal portion of the first shaft 24.

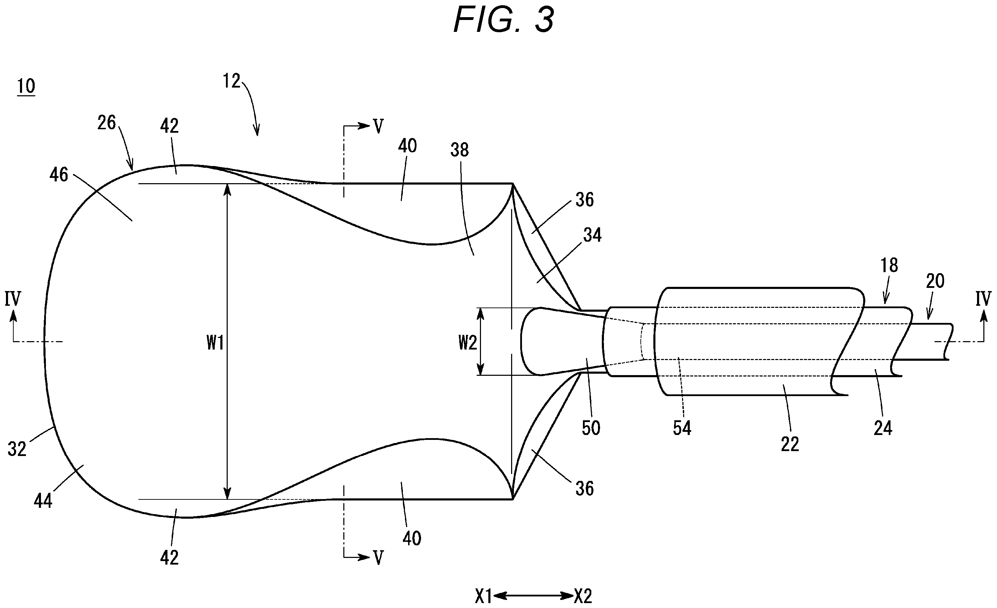

As illustrated in Fig. 3, the support body 32 extends in a distal end direction from the joint 30 (see Fig. 4). The support body 32 includes a proximal-end support portion 34, a pair of first protrusions 36, an intermediate support portion 38, a pair of second protrusions 40, a pair of third protrusions 42, and a distal-end support portion 44.

The proximal-end support portion 34 extends roughly along an axis Ax of the first shaft 24 from a distal end of the joint 30 in the distal end direction (see Fig. 4). The proximal-end support portion 34 is formed to be wider in its extending direction. In other words, both sides of the proximal-end support portion 34 in a width direction are tapered toward the joint 30.

In Figs. 2 and 3, the pair of first protrusions 36 protrude upward (in a direction of arrow Y2) from both the sides of the proximal-end support portion 34. The intermediate support portion 38 extends from a distal end of the proximal-end support portion 34 in the distal end direction at an angle relative to the proximal-end support portion 34 so as to approach the axis Ax of the first shaft 24 (see Fig. 4). The intermediate support portion 38 is formed with a substantially constant width over the entire length. Note that the intermediate support portion 38 may be formed to be wider or narrower in the distal end direction.

The pair of second protrusions 40 are connected to distal ends of the pair of first protrusions 36. The pair of second protrusions 40 protrude upward from both sides of the intermediate support portion 38 in the width direction and inward in the width direction of the intermediate support portion 38. The pair of third protrusions 42 are connected to distal ends of the pair of second protrusions 40. The pair of third protrusions 42 protrude upward from both the sides of the intermediate support portion 38 in the width direction and outward in the width direction of the intermediate support portion 38.

The distal-end support portion 44 is connected to a distal end of the intermediate support portion 38 and distal ends of the pair of third protrusions 42. The distal-end support portion 44 protrudes in an arc shape in the distal end direction. In other words, the distal-end support portion 44 is formed to be narrower in the distal end direction.

The support body 32 includes a support surface 46 facing upward. Specifically, the support surface 46 is formed by an upper surface of the intermediate support portion 38, an upper surface of the distal-end support portion 44, and upper surfaces of the pair of third protrusions 42. A lubricant may be applied to the support surface 46 so as to allow a pressing portion 50 (to be described later) of the second carrier member 20 to smoothly slide on the support surface 46.

A part of the support surface 46 formed by the upper surface of the intermediate support portion 38 and the upper surface of the distal-end support portion 44 is a flat surface (see Fig. 4). Note that the part of the support surface 46 formed by the upper surface of the intermediate support portion 38 and the upper surface of the distal-end support portion 44 may be a curved surface that curves in a direction opposite to the direction that the support surface 46 faces.

As illustrated in Fig. 2, the second carrier member 20 includes a second shaft 54, the pressing portion 50, and a connector 52. The second shaft 54 is a circular tube member having a second lumen 57. The second lumen 57 opens at a distal end (end in the direction of arrow X1) of the second shaft 54 and opens at a proximal end (end in the direction of arrow X2) of the second shaft 54.

The second shaft 54 is longer in the axial direction than the first shaft 24. The second shaft 54 is inserted into the first lumen 28 of the first shaft 24 (see Figs. 1 and 4). In other words, a distal portion of the second shaft 54 protrudes in the distal end direction relative to a distal-end opening of the first shaft 24. A proximal portion of the second shaft 54 protrudes in the proximal end direction relative to a proximal-end opening of the first shaft 24 (see Fig. 1).

The second shaft 54 is configured to follow the shape (angle) of the support portion 26. As the constituent material of the second shaft 54, for example, a material more flexible than the constituent material of the first shaft 24 is selected. Specifically, examples of the constituent material of the second shaft 54 include a polyamide elastomer, a polyester elastomer, a polyurethane elastomer, polyvinyl chloride, polybutadiene, a silicone rubber, and a metal coil (including a composite with a resin). The second shaft 54 is flexible. Note that in a case where the first shaft 24 includes the flexible tube portion, the second shaft 54 bends along the bent shape of the first shaft 24. The pressing portion 50 is attached to the distal portion of the second shaft 54.

As illustrated in Fig. 4, an insertion hole 58, a pressing surface 64, and a supply hole 66 are formed in the pressing portion 50. The distal portion of the second shaft 54 is inserted into the insertion hole 58. The distal portion of the second shaft 54 is thermally fused to an inner surface forming the insertion hole 58. Note that the distal portion of the second shaft 54 may be joined to the inner surface forming the insertion hole 58 with an adhesive. In Fig. 3, the pressing portion 50 is formed to be wider in the distal end direction when viewed from above. The pressing portion 50 is formed in a trapezoidal shape when viewed from above.

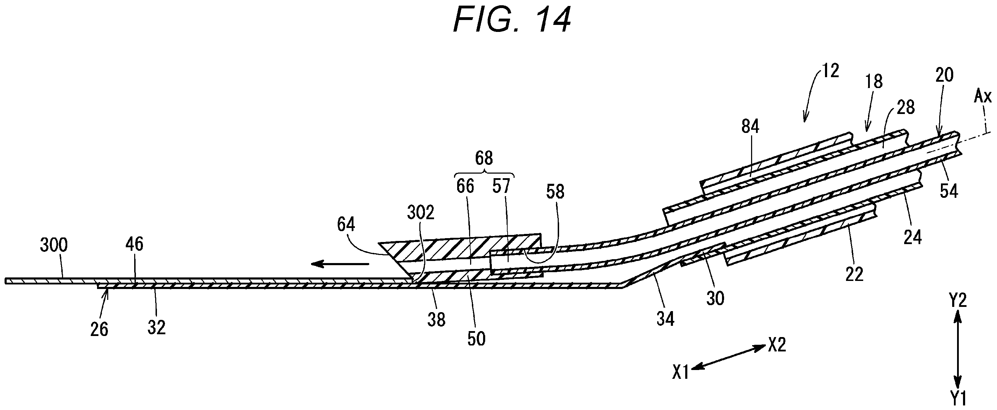

In Fig. 4, the pressing surface 64 is provided on a distal end surface of the pressing portion 50. The pressing surface 64 presses an outer edge surface 302 (see Fig. 14) of the medical sheet 300 in the distal end direction. The pressing surface 64 extends upward with an inclination in the distal end direction (the direction of arrow X1). The pressing surface 64 includes a flat surface.

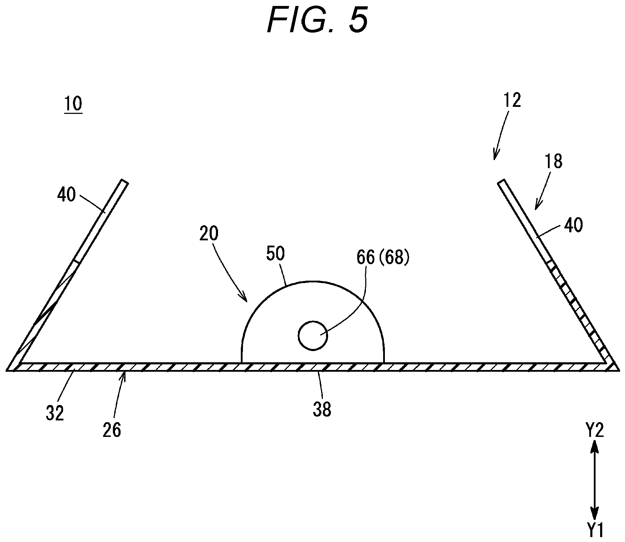

As illustrated in Figs. 4 and 5, the supply hole 66 communicates with the second lumen 57 of the second shaft 54 and opens to the pressing surface 64. The supply hole 66 and the second lumen 57 communicate with each other to form a liquid supply flow path 68. The liquid supply flow path 68 guides, toward a distal end of the pressing portion 50, a liquid for preventing the medical sheet 300 from drying out. As the liquid, for example, a physiological saline solution is used.

In Fig. 2, the connector 52 is attached to the proximal portion of the second shaft 54. The connector 52 is configured as a so-called straight connector. The connector 52 includes a connection port 82 to which a liquid supply instrument (not illustrated) is detachably attached.

In Figs. 1 and 2, the outer cylinder 22 is a cylindrical member having a lumen 84. The lumen 84 opens at a distal end (end in the direction of arrow X1) of the outer cylinder 22 and opens at a proximal end (end in the direction of arrow X2) of the outer cylinder 22. The outer cylinder 22 is flexible. Examples of the constituent material of the outer cylinder 22 are the same as the examples of the constituent material of the first shaft 24 described above. Note that in a case where the first shaft 24 includes the flexible tube portion, the outer cylinder 22 bends along the bent shape of the first shaft 24. Further, in the present embodiment, the outer cylinder 22 may include the flexible tube portion as described above. In this case, the first shaft 24 need not include the flexible tube portion.

The first shaft 24 is inserted into the lumen 84 of the outer cylinder 22. The outer cylinder 22 is shorter in the axial direction than the first shaft 24. In Figs. 3 and 4, an inner diameter D1 of the outer cylinder 22 is smaller than the width W1 of the intermediate support portion 38. The width W1 of the intermediate support portion 38 is substantially identical to a circumferential length of an inner surface of the outer cylinder 22 so as to allow the support portion 26 to be retracted into the outer cylinder 22 with the support portion 26 curled into a cylinder along a circumferential direction of an inner peripheral surface of the outer cylinder 22. Note that the width W1 may be shorter or longer than the circumferential length of the inner surface of the outer cylinder 22 as long as the support portion 26 can be retracted into the outer cylinder 22. A maximum width W2 of the pressing portion 50 is smaller than an inner diameter D1 of the outer cylinder 22 and larger than an outer diameter of the second shaft 54. It is therefore possible to make the pressing portion 50 relatively wide and retract the pressing portion 50 into the outer cylinder 22 with ease. The maximum width W2 of the pressing portion 50 is smaller than the width W1 of the intermediate support portion 38 and larger than the outer diameter of the second shaft 54.

In Figs. 2 and 4, a distal end surface of the outer cylinder 22 extends in a direction orthogonal to the axial direction of the outer cylinder 22. Note that the distal end surface of the outer cylinder 22 may extend upward with an inclination in the proximal end direction. In this case, for example, in thoracoscopic surgery, the outer cylinder 22 is easily inserted into an incision 409 (Fig. 12) of a chest 408. Further, the angle of the distal end surface of the outer cylinder 22 serves as a guide for a surgeon to recognize the upper surface of the support portion 26.

As illustrated in Figs. 1 and 2, the endoscope 14 includes a long endoscope main body 86. A distal portion of the endoscope main body 86 is fixed to an outer peripheral surface of the outer cylinder 22 by the fixing member 16. An objective lens 88 provided on a distal end surface of the endoscope main body 86 is oriented toward the distal end of the outer cylinder 22 (in the direction of arrow X1). The distal portion of the endoscope main body 86 is fixed to an intermediate portion of the outer cylinder 22 in the axial direction. Note that the distal portion of the endoscope main body 86 may be fixed to a distal portion of the outer cylinder 22.

The fixing member 16 includes, for example, a fixing cylinder 87 and a fixing tube 89. The fixing cylinder 87 includes, for example, a hard resin material. The endoscope main body 86 can be inserted into a lumen of the fixing cylinder 87. The fixing cylinder 87 is disposed along a longitudinal direction of the outer cylinder 22. The fixing tube 89 is a tube for fixing the fixing cylinder 87 at a predetermined position of the outer cylinder 22. The fixing tube 89 is, for example, a heat-shrinkable tube. Note that the outer cylinder 22 and the fixing cylinder 87 may be integrally molded. Note that how to fix the distal portion of the endoscope main body 86 to the outer cylinder 22 may be determined as desired.

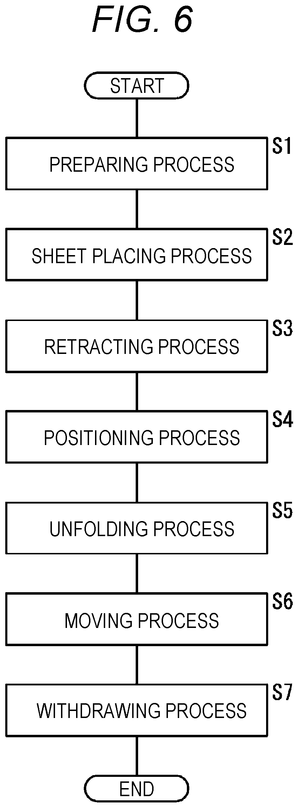

Next, a transfer method for transferring the medical sheet 300 to a treatment site of a living body will be described. Specifically, as illustrated in Figs. 12 to 15, the transfer method for transferring the medical sheet 300 to the recipient site 402 of the heart 400 (the treatment site of the living body) during thoracoscopic surgery will be described. As illustrated in Fig. 6, the transfer method according to the present embodiment includes a preparing process, a sheet placing process, a retracting process, a positioning process, an unfolding process, a moving process, and a withdrawing process.

First, in the preparing process (step S1), the transfer instrument 10 according to the present embodiment described above is prepared. The transfer instrument 10 is brought into an initial state as illustrated in Fig. 1. That is, the support portion 26 protrudes in the distal end direction from a distal-end opening of the outer cylinder 22. At this time, the pressing portion 50 is positioned on the support surface 46. Further, a proximal portion of the pressing portion 50 is in the first lumen 28 of the first shaft 24.

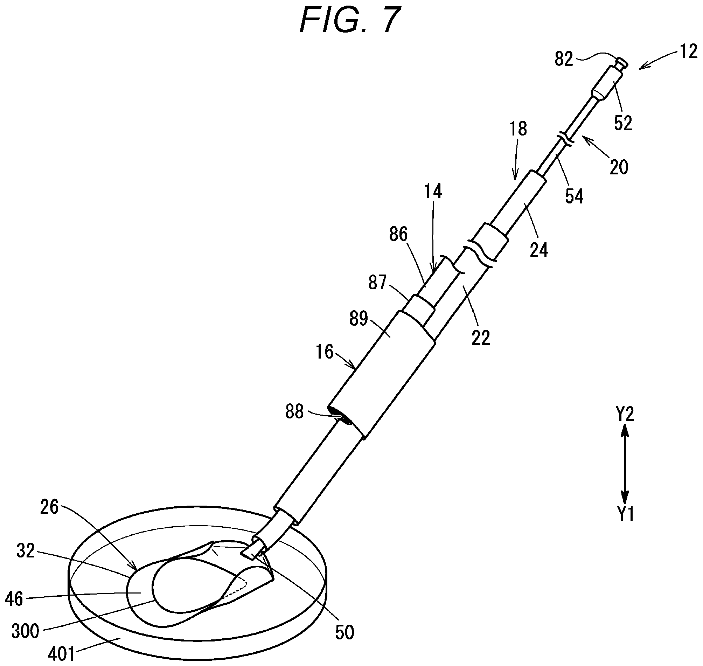

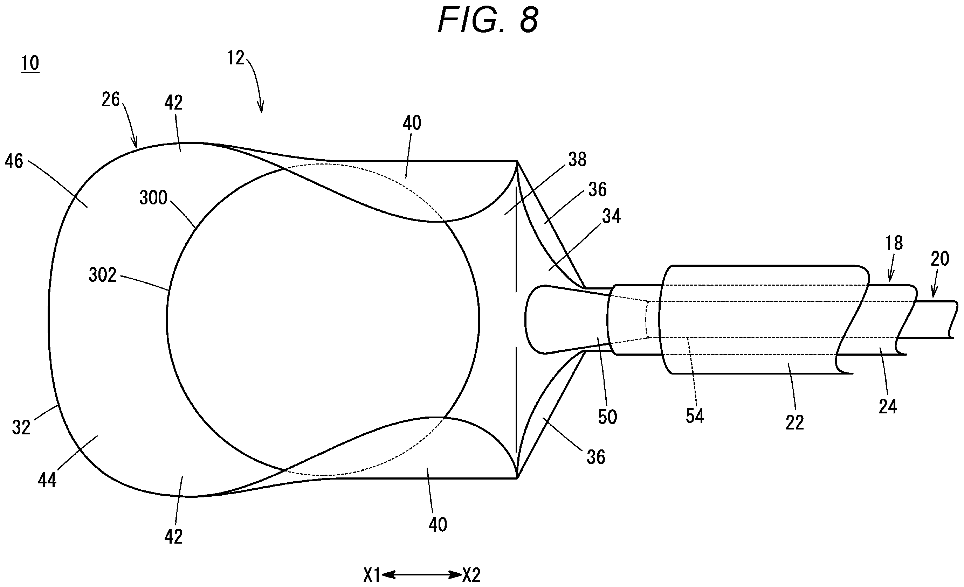

Next, in the sheet placing process (step S2), as illustrated in Figs. 7 and 8, the medical sheet 300 cultured in a Petri dish 401 is placed on the support surface 46. The pair of second protrusions 40 prevent the medical sheet 300 from moving (becoming misaligned) in the width direction of the intermediate support portion 38 with the medical sheet 300 placed on the support surface 46.

Subsequently, in the retracting process (step S3 in Fig. 6), the medical sheet 300 is retracted into the outer cylinder 22 together with the support portion 26. Specifically, the first shaft 24 of the first carrier member 18 and the second shaft 54 of the second carrier member 20 are moved together in the proximal end direction relative to the outer cylinder 22.

Accordingly, the proximal-end support portion 34 is pulled in the proximal end direction through the distal-end opening of the outer cylinder 22. At this time, when both the tapered sides of the proximal-end support portion 34 come into contact with the distal end surface of the outer cylinder 22, a force acts on the proximal-end support portion 34 to cause the proximal-end support portion 34 to curl along the circumferential direction of the outer cylinder 22. Therefore, the proximal-end support portion 34 is smoothly pulled into the outer cylinder 22 while curling.

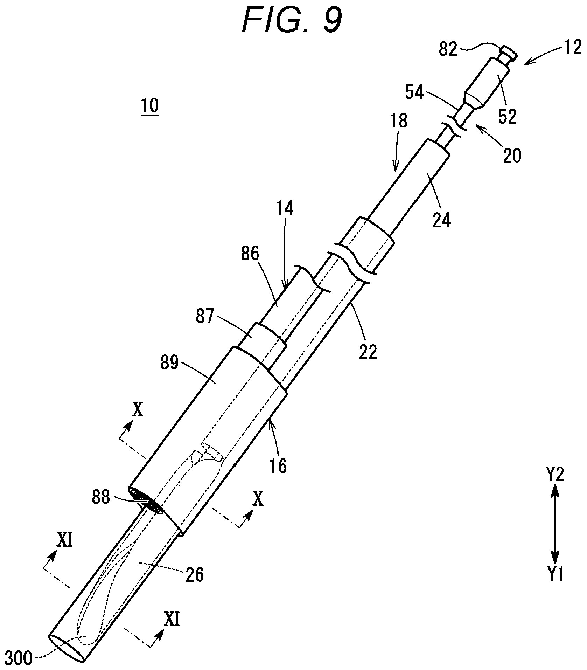

When the proximal-end support portion 34 becomes deformed, a force acts on the intermediate support portion 38 to cause the intermediate support portion 38 to curl along the circumferential direction of the outer cylinder 22, so that the intermediate support portion 38 is pulled into the outer cylinder 22 while curling. At this time, since the width W1 of the intermediate support portion 38 is substantially identical to the circumferential length of the inner surface of the outer cylinder 22, the intermediate support portion 38 is deformed into a cylindrical shape along the inner surface of the outer cylinder 22. The medical sheet 300 is inserted into the outer cylinder 22 with the medical sheet 300 deformed into a shape corresponding to the shape of the support body 32. As illustrated in Fig. 9, when the support portion 26 is entirely inserted into the outer cylinder 22, the retracting process is complete.

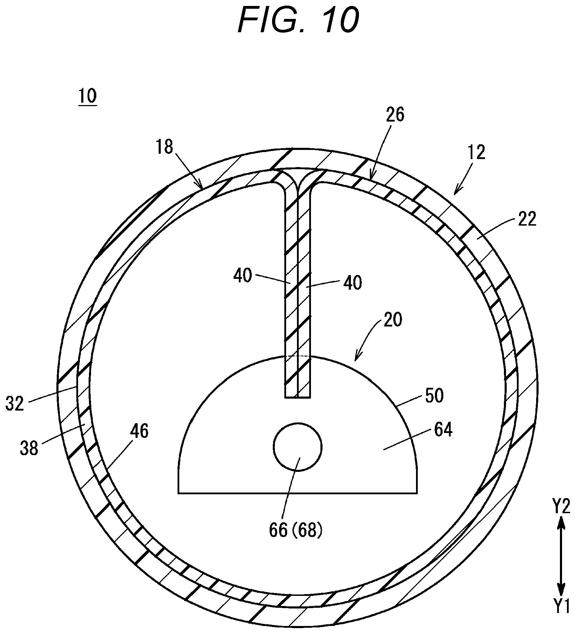

In a retracted state where the support portion 26 and the medical sheet 300 are retracted in the outer cylinder 22, as illustrated in Fig. 10, the pair of second protrusions 40 are positioned in the distal end direction relative to the pressing surface 64 of the pressing portion 50 with the pair of second protrusions 40 in contact with each other. This restricts the movement of the second carrier member 20 in the distal end direction relative to the first carrier member 18. That is, the movement of the second shaft 54 in the distal end direction relative to the first shaft 24 is restricted. Therefore, even in a case where the user pushes by mistake the second shaft 54 in the distal end direction relative to the first shaft 24, it is possible to prevent the medical sheet 300 from protruding from the distal-end opening of the outer cylinder 22. Further, the second shaft 54 can be prevented from rotating in the circumferential direction relative to the first shaft 24.

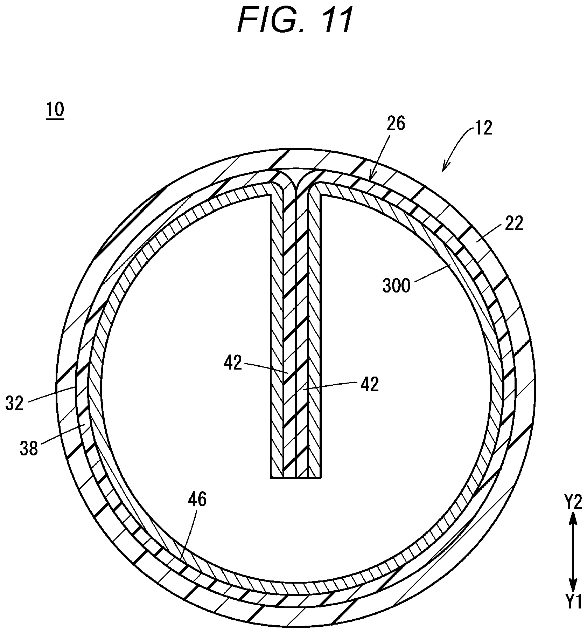

In the retracted state, as illustrated in Fig. 11, the pair of third protrusions 42 extend downward (in the direction of arrow Y1) from an upper end of the outer cylinder 22 with the pair of third protrusions 42 in contact with each other. Protruding end surfaces of the pair of third protrusions 42 are separated from the medical sheet 300. The medical sheet 300 is deformed along the shape of the support portion 26. That is, this prevents the medical sheet 300 from being folded over in the outer cylinder 22.

Subsequently, in the positioning process (step S4 in Fig. 6), as illustrated in Fig. 12, the transfer instrument 10 is inserted into a chest cavity 410 through the incision 409 of the chest 408. At this time, the distal end of the transfer instrument 10 is positioned near the recipient site 402 of the heart 400, and the distal end of the endoscope 14 is positioned in the chest cavity 410. Note that before the transfer instrument 10 is inserted into the chest cavity 410, a liquid supply instrument (not illustrated) may be connected to the connection port 82 of the connector 52 to introduce a liquid (for example, a physiological saline solution). The liquid introduced from the connection port 82 is guided to the medical sheet 300 through the liquid supply flow path 68 (the second lumen 57 and the supply hole 66). This makes it possible to prevent the medical sheet 300 from drying out.

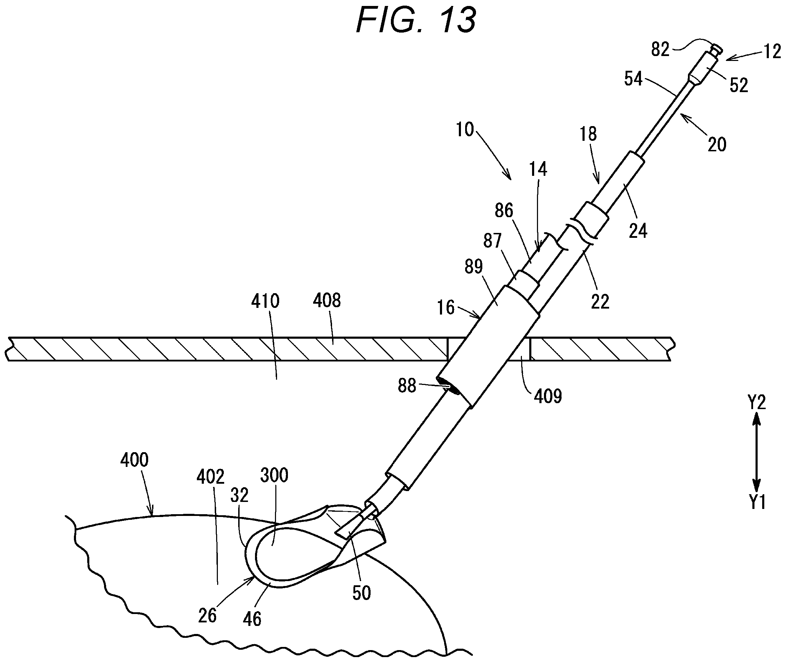

Subsequently, in the unfolding process (step S5 in Fig. 6), as illustrated in Fig. 13, the support portion 26 and the medical sheet 300 are unfolded. Specifically, in the unfolding process, the first shaft 24 and the second shaft 54 are moved in the distal end direction relative to the outer cylinder 22. Accordingly, the support portion 26 that has moved out of the distal-end opening of the outer cylinder 22 returns to the original shape due to its restoring force. When the support portion 26 is unfolded, the medical sheet 300 unrolls flat.

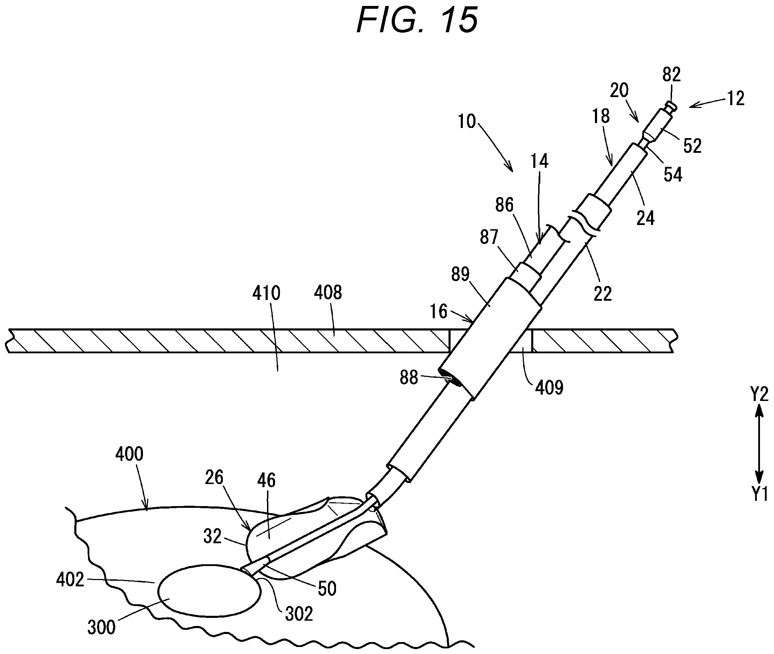

Next, in the moving process (step S6 in Fig. 6), as illustrated in Figs. 14 and 15, moving the second shaft 54 in the distal end direction relative to the first shaft 24 causes the pressing surface 64 to press, in the distal end direction, a part facing the proximal end direction of the outer edge surface 302 of the medical sheet 300 placed on the support surface 46. At this time, the pressing portion 50 slides on the support surface 46 in the distal end direction. The medical sheet 300 pressed by the pressing portion 50 slides on the support surface 46 in the distal end direction while maintaining its original shape. It is therefore possible to move the medical sheet 300 smoothly from the support surface 46 to the recipient site 402 of the heart 400.

Subsequently, in the withdrawing process (step S7 in Fig. 6), the transfer instrument 10 is withdrawn from the chest 408 with the support portion 26 and the pressing portion 50 retracted in the outer cylinder 22.

The transfer instrument 10 according to the present embodiment has the following effects.

According to the present embodiment, it is possible to slide, by causing the pressing surface 64 of the pressing portion 50 to press, in the distal end direction, the outer edge surface 302 of the medical sheet 300 placed on the support surface 46 of the first carrier member 18, the medical sheet 300 from the support surface 46 to the recipient site 402 of the heart 400. It is therefore possible to efficiently transfer the medical sheet 300 to the recipient site 402.

The first shaft 24 is formed in a tubular shape. The second shaft 54 is inserted into the first lumen 28 of the first shaft 24 with the second shaft 54 movable along the axial direction of the first shaft 24.

Such a configuration can make the transfer instrument 10 compact as compared with a configuration where the second shaft 54 is disposed outside the first shaft 24.

The pressing portion 50 slides on the support surface 46 when the outer edge surface 302 of the medical sheet 300 is pressed in the distal end direction by the pressing surface 64.

Such a configuration allows the pressing surface 64 to efficiently press the outer edge surface 302 of the medical sheet 300 placed on the support surface 46.

The second carrier member 20 includes the liquid supply flow path 68 through which a liquid is supplied to the medical sheet 300 placed on the support surface 46.

Such a configuration allows a liquid to be supplied to the medical sheet 300 through the liquid supply flow path 68, so that it is possible to prevent the medical sheet 300 from drying out.

The pressing surface 64 extends upward with an inclination in the distal end direction.

Such a configuration can prevent the medical sheet 300 from running on to the pressing surface 64.

The pressing portion 50 is formed to be wider in the distal end direction when viewed from above.

Such a configuration allows an increase in the area of the pressing surface 64 with ease.

The transfer instrument 10 includes the outer cylinder 22 through which the first shaft 24 is inserted. The support portion 26 is flexible and formed in a sheet shape. The support portion 26 is formed to be wider than the inner diameter D1 of the outer cylinder 22. When the first shaft 24 and the second shaft 54 are moved in the proximal end direction relative to the outer cylinder 22, the support portion 26 and the medical sheet 300 are retracted into the outer cylinder 22 with the support portion 26 and the medical sheet 300 undergoing curved deformation along the circumferential direction of the outer cylinder 22. The support portion 26 and the medical sheet 300 retracted in the outer cylinder 22 are unfolded when the first shaft 24 and the second shaft 54 are moved in the distal end direction relative to the outer cylinder 22 to be exposed from the outer cylinder 22.

Such a configuration allows, in thoracoscopic surgery (body cavity surgery), the outer cylinder 22 to be inserted into the chest cavity 410 (body cavity) from the incision 409 of the chest 408 with the support portion 26 and the medical sheet 300 retracted in the outer cylinder 22. Note that the body cavity includes the chest cavity 410 and the abdominal cavity. Further, in the chest cavity 410, when the support portion 26 in the retracted state is exposed from the outer cylinder 22, the support portion 26 and the medical sheet 300 can be unfolded. It is therefore possible to make a small incision in the chest 408 of the patient and transfer the medical sheet 300 to the recipient site 402 through the small incision, so that it is possible to achieve low invasion as compared with thoracotomy. Furthermore, in thoracoscopic surgery, the medical sheet 300 having a diameter larger than the inner diameter D1 of the outer cylinder 22 can be positioned adjacent to the recipient site 402 (the treatment site of the living body) of the heart 400.

The maximum width W2 of the pressing portion 50 is smaller than the width of the support portion 26 (the width W1 of the intermediate support portion 38) and larger than the outer diameter of the second shaft 54.

Such a configuration can make the pressing surface 64 relatively wide.

The support portion 26 includes the pair of second protrusions 40 protruding upward from both sides of the support surface 46 in the width direction.

Such a configuration allows the pair of second protrusions 40 to prevent the medical sheet 300 from falling outside of the support body 32.

The transfer instrument 10 further includes the endoscope 14 that captures an image of the support portion 26, the pressing portion 50, and the medical sheet 300.

Such a configuration makes it possible to check, with the endoscope 14, how the medical sheet 300 is transferred to the recipient site 402.

### (First modification)

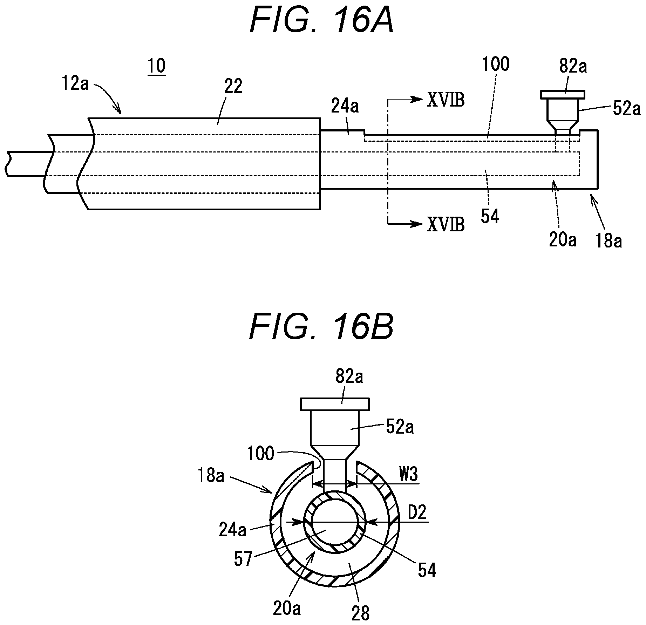

As illustrated in Figs. 16A and 16B, the transfer instrument 10 may include an instrument body 12a according to a first modification. Note that, in the present modification, the same components as those of the above-described instrument body 12 are denoted by the same reference numerals, and the detailed description of the same components will be omitted. The same applies to an instrument body 12b according to a second modification to be described later. The instrument body 12a includes a first carrier member 18a, a second carrier member 20a, and the outer cylinder 22.

The first carrier member 18a includes a first shaft 24a and the support portion 26 (see Fig. 2). An insertion slit 100 is formed in a proximal portion of the first shaft 24a. The insertion slit 100 communicates with a first lumen 28 of the first shaft 24a. In other words, the insertion slit 100 extends through a peripheral wall of the first shaft 24a in the radial direction. The insertion slit 100 extends along the axial direction of the outer cylinder 22. A width W3 of the insertion slit 100 is smaller than an outer diameter D2 of the second shaft 54 to be described later (see Fig. 16B).

The second carrier member 20a includes the second shaft 54 and a connector 52a. The proximal end of the second shaft 54 is sealed so as to prevent the second lumen 57 from communicating with the outside. The connector 52a extends through the insertion slit 100 and extends radially outward from the proximal portion of the second shaft 54. The connector 52a includes a connection port 82a exposed to the outside of the first shaft 24a.

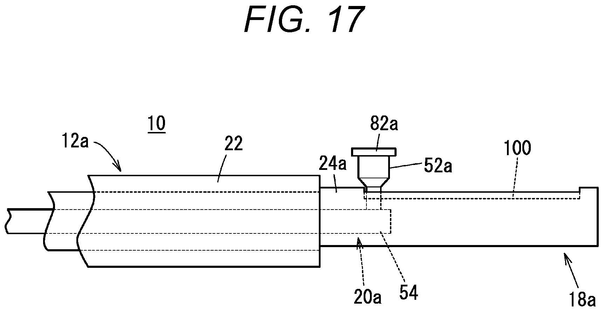

As illustrated in Fig. 17, such a configuration causes the connector 52a to move through the insertion slit 100 in the distal end direction when the second carrier member 20a is moved in the distal end direction. In this case, the overall length of the transfer instrument 10 can be shortened.

### (First configuration example)

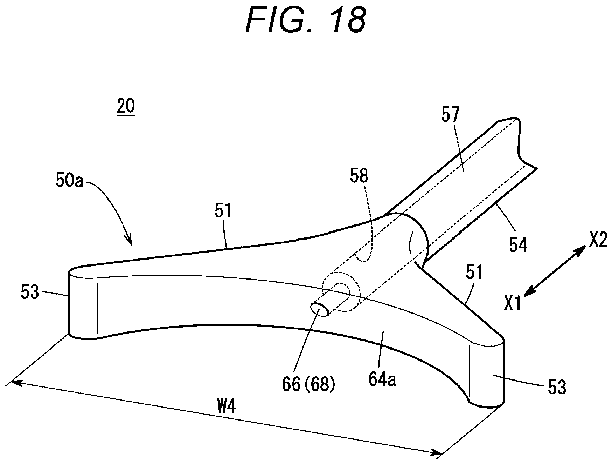

As illustrated in Fig. 18, the second carrier member 20 may include a pressing portion 50a according to a first configuration example instead of the pressing portion 50 described above.

The pressing portion 50a is formed to be wider in the distal end direction. Specifically, the pressing portion 50a extends outward in the width direction while extending in the distal end direction. Both side surfaces 51 in the width direction of the pressing portion 50a are flat surfaces tapered in the proximal end direction (toward the second shaft 54). Note that each of the side surfaces 51 may be a curved surface curved in an arc shape outward in the width direction. Both end portions 53 on the outer sides in the width direction of the pressing portion 50a are curved in an arc shape (semicircular shape) when viewed from above. The pressing portion 50a is formed symmetrically about the axis of the second shaft 54. A distal portion of the pressing portion 50a is provided with a pressing surface 64a facing the distal end direction.

The pressing surface 64a is curved in an arc shape in the proximal end direction when viewed from above. In other words, the pressing surface 64a is curved in an arc shape in the proximal end direction to follow the shape of the outer periphery of the medical sheet 300. The pressing surface 64a extends upward with an inclination in the distal end direction. That is, the pressing surface 64a is at an angle relative to a plane orthogonal to the axis of the second shaft 54.

A maximum width W4 of the pressing portion 50a (a width of the distal end of the pressing portion 50a) is larger than the inner diameter D1 of the outer cylinder 22 (see Fig. 4). The pressing portion 50a has flexibility so as to be retractable into the outer cylinder 22. The pressing portion 50a includes a soft resin material. The maximum width W4 of the pressing portion 50a is smaller than the width W1 of the intermediate support portion 38 and larger than the outer diameter of the second shaft 54. On the pressing surface 64a, the supply hole 66 through which a liquid is supplied from the pressing portion 50a in the distal end direction opens.

The pressing portion 50a according to the present configuration example produces the same effect as the effect produced by the pressing portion 50 described above. That is, the maximum width W4 of the pressing portion 50a is smaller than the width of the support portion 26 (the width W1 of the intermediate support portion 38) and larger than the outer diameter of the second shaft 54. It is therefore possible to make the pressing surface 64a relatively wide.

The pressing portion 50a is formed to be wider in the distal end direction when viewed from above.

Such a configuration can make the pressing surface 64a wider.

The pressing surface 64a is curved in an arc shape in the proximal end direction when viewed from above.

Such a configuration allows, for example, even in a case where the medical sheet 300 is formed in a circular shape, the pressing surface 64a to reliably press the outer edge surface 302 of the medical sheet 300.

The pressing portion 50a is flexible. The maximum width W4 (the width of the pressing surface 64a) of the pressing portion 50a is larger than the inner diameter D1 of the outer cylinder 22.

Such a configuration can make the pressing surface 64a wider with the pressing portion 50a retractable into the outer cylinder 22.

In the present configuration example, the pressing portion 50a may include a hard resin material. In this case, the maximum width W4 of the pressing portion 50a is smaller than the inner diameter D1 of the outer cylinder 22 so as to make the pressing portion 50a retractable into the outer cylinder 22. Further, the pressing portion 50a may be integrally molded with the second shaft 54. The supply hole 66 need not be formed in the pressing portion 50a.

### (Second configuration example)

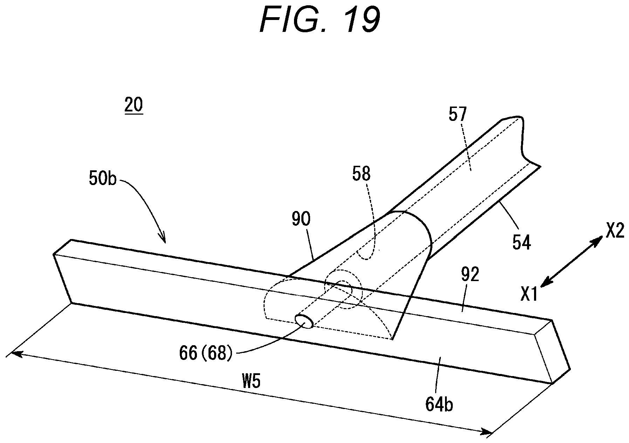

As illustrated in Fig. 19, the second carrier member 20 may include a pressing portion 50b according to a second configuration example instead of the pressing portion 50 described above.

The pressing portion 50b includes a connection portion 90 and a pressing body 92. The connection portion 90 has the same configuration as the configuration of the pressing portion 50 described above. That is, the connection portion 90 is formed to be wider in the distal end direction. The pressing body 92 is attached to a flat distal end surface of the connection portion 90. The pressing body 92 is a flat plate portion extending in one direction. The pressing body 92 includes a pressing surface 64b facing the distal end direction.

The pressing surface 64b extends upward with an inclination in the distal end direction. That is, the pressing surface 64b is at an angle relative to a plane orthogonal to the axis of the second shaft 54. The pressing surface 64b is a flat surface extending along a direction (width direction) orthogonal to the axis of the second shaft 54. A maximum width W5 of the pressing portion 50b (a width of a distal end of the pressing portion 50b) is larger than the inner diameter D1 of the outer cylinder 22 (see Fig. 4). The pressing portion 50b has flexibility so as to be retractable into the outer cylinder 22. The pressing portion 50b includes a soft resin material. On the pressing surface 64b, the supply hole 66 through which a liquid is supplied from the pressing portion 50b in the distal end direction opens.

The pressing portion 50b according to the present configuration example produces the same effect as the effect produced by the pressing portion 50 described above.

The pressing portion 50b is flexible. The maximum width W5 of the pressing portion 50b is larger than the inner diameter D1 of the outer cylinder 22.

Such a configuration can make the pressing surface 64b wider with the pressing portion 50b retractable into the outer cylinder 22.

In the present configuration example, the pressing portion 50b may include a hard resin material. In this case, the maximum width W5 of the pressing portion 50b is smaller than the inner diameter D1 of the outer cylinder 22 so as to make the pressing portion 50b retractable into the outer cylinder 22. Further, the pressing portion 50b may be integrally molded with the second shaft 54. The supply hole 66 need not be formed in the pressing portion 50b.

### (Third configuration example)

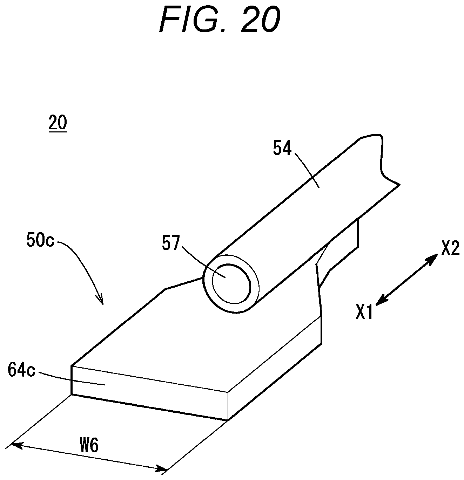

As illustrated in Fig. 20, the second carrier member 20 may include a pressing portion 50c according to a third configuration example instead of the pressing portion 50 described above.

The pressing portion 50c is a plate-shaped portion attached to the outer peripheral surface of the distal portion of the second shaft 54. Specifically, the pressing portion 50c is thermally fused to the second shaft 54. Note that the pressing portion 50c may be joined to the second shaft 54 with an adhesive or the like.

The pressing portion 50c protrudes in the distal end direction relative to the distal end of the second shaft 54. A distal portion of the pressing portion 50c is provided with a pressing surface 64c facing the distal end direction. The pressing surface 64c is a flat surface. A maximum width W6 of the pressing portion 50c (a width of a distal end of the pressing portion 50c) is smaller than the inner diameter D1 of the outer cylinder 22 (see Fig. 4). The maximum width W6 is larger than the outer diameter of the second shaft 54. The pressing portion 50c includes a hard resin material. Note that the pressing portion 50c may include a soft resin material. The distal end of the second shaft 54 (the distal-end opening of the second lumen 57) is positioned above the pressing portion 50c.

In the present configuration example, the pressing portion 50c does not include the supply hole 66 described above. A liquid guided from the second lumen 57 (liquid supply flow path 68) of the second shaft 54 flows on the upper surface of the pressing portion 50c and is supplied to the medical sheet 300 positioned in the distal end relative to the pressing surface 64c.

The pressing portion 50c according to the present configuration example produces the same effect as the effect produced by the pressing portion 50c described above.

In the present configuration example, since the plate-shaped pressing portion 50c is attached to the outer peripheral surface of the distal portion of the second shaft 54, the configuration of the second shaft 54 can be made simple.

### (Second modification)

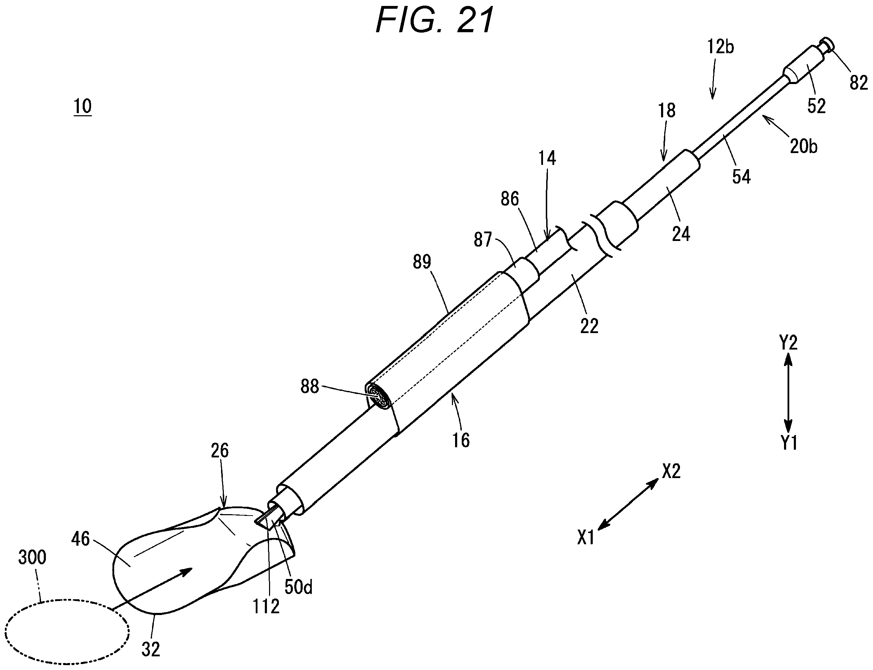

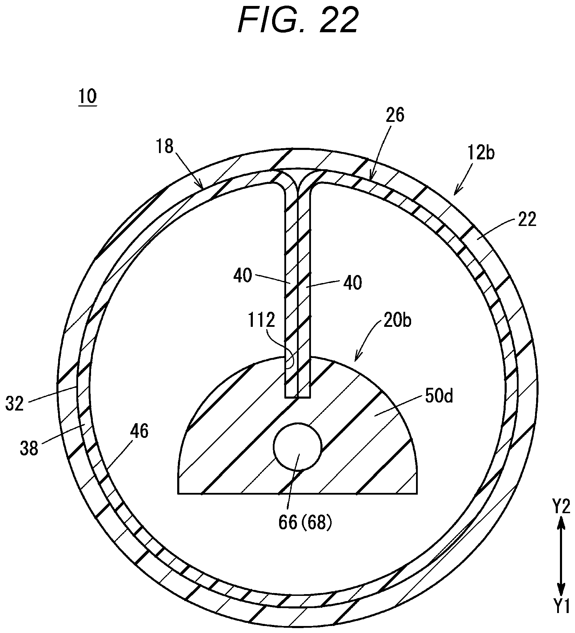

As illustrated in Figs. 21 and 22, the transfer instrument 10 may include the instrument body 12b according to the second modification. The instrument body 12b includes the first carrier member 18, a second carrier member 20b, and the outer cylinder 22.

The second carrier member 20b includes the second shaft 54, a pressing portion 50d, and the connector 52. A retaining slit 112 is formed in an upper surface of the pressing portion 50d. The retaining slit 112 extends over the entire length of the pressing portion 50d. The retaining slit 112 opens upward.

In the present modification, in the retracted state where the support portion 26, the pressing portion 50d, and the medical sheet 300 are retracted in the outer cylinder 22, the pair of second protrusions 40 are in the retaining slit 112 of the pressing portion 50d with the pair of second protrusions 40 in contact with each other, as illustrated in Fig. 22. This restricts the movement of the second carrier member 20b relative to the first carrier member 18. That is, the movement of the second shaft 54 along the axial direction relative to the first shaft 24 is restricted. Therefore, even in a case where the user presses, by mistake, the second shaft 54 in the distal end direction relative to the first shaft 24, it is possible to prevent the pressing portion 50d and the medical sheet 300 from protruding from the distal-end opening of the outer cylinder 22. Further, the second shaft 54 can be prevented from rotating in the circumferential direction relative to the first shaft 24.

The retaining slit 112 into which the pair of second protrusions 40 are inserted in the retracted state is formed in the pressing portion 50d.

Such a configuration allows the pressing portion 50d to hold the support portion 26 in the retracted state. It is therefore possible to prevent, even in a case where the second shaft 54 is pushed by mistake in the distal end direction relative to the first shaft 24, the medical sheet 300 from protruding from the outer cylinder 22.

The transfer instrument 10 according to the present embodiment may have a configuration obtained by combining the configurations according to the first modification and the second modification as desired. The endoscope 14 may be disposed in the lumen 84 of the outer cylinder 22. Further, the endoscope 14 may be provided separately from the instrument bodies 12, 12a, and 12b. Note that the transfer instrument 10 need not include the endoscope 14.

The transfer instrument 10 described above may include a lock mechanism that restricts relative movement of the first shafts 24 and 24a and the second shaft 54. The lock mechanism can be switched between a locked state where the relative movement of the first shafts 24 and 24a and the second shaft 54 along the axial direction of the first shafts 24 and 24a is restricted and a released state where the restriction is removed. In this case, it is possible to move, by bringing the lock mechanism into the locked state, the first shafts 24 and 24a and the second shaft 54 together relative to the outer cylinder 22 in the retracting process and the positioning process. That is, it is possible to prevent an erroneous operation of moving the second shaft 54 relative to the first shafts 24 and 24a in the retracting process and the positioning process.

The transfer instrument 10 may be used for laparoscopic surgery rather than thoracoscopic surgery. Further, the transfer instrument 10 may be used for thoracotomy or laparotomy.

The present embodiment discloses a sheet transfer method for transferring, with a transfer instrument, a medical sheet to a treatment site of a living body, the transfer instrument including a first carrier member including a first shaft and a support portion, the support portion being provided at a distal portion of the first shaft and including a support surface, and a second carrier member including a second shaft and a pressing portion, the second shaft extending along an axial direction of the first shaft, the pressing portion being provided at a distal portion of the second shaft, and in the sheet transfer method, moving the second shaft in a distal end direction relative to the first shaft causes a pressing surface of the pressing portion to press, in the distal end direction, an outer edge surface of the medical sheet placed on the support surface.

Note that the present invention is not limited to the disclosure, and various configurations can be adopted without departing from the gist of the present invention.

## Claims

1. A transfer instrument that transfers a medical sheet to a treatment site of a living body, the transfer instrument comprising:
a first carrier member including a first shaft and a support portion, the support portion being provided at a distal portion of the first shaft and including a support surface on which the medical sheet is placed; and
a second carrier member including a second shaft and a pressing portion, the second shaft extending along an axial direction of the first shaft, the pressing portion being provided at a distal portion of the second shaft, wherein
the second carrier member is movable relative to the first carrier member so as to allow a pressing surface of the pressing portion to press, in a distal end direction, an outer edge surface of the medical sheet placed on the support surface.

2. The transfer instrument according to claim 1, wherein
the first shaft is formed in a tubular shape, and
the second shaft is inserted into a lumen of the first shaft so as to be movable along the axial direction of the first shaft.

3. The transfer instrument according to claim 1, wherein
the pressing portion slides on the support surface when the outer edge surface of the medical sheet is pressed in the distal end direction by the pressing surface.

4. The transfer instrument according to claim 1, wherein
the second carrier member includes a liquid supply flow path through which a liquid is supplied to the medical sheet placed on the support surface.

5. The transfer instrument according to claim 1, wherein
the pressing surface extends upward with an inclination in the distal end direction.

6. The transfer instrument according to claim 1, wherein
the pressing portion is formed to be wider in the distal end direction when viewed from above.

7. The transfer instrument according to claim 1, wherein
the pressing surface is curved in an arc shape in a proximal end direction when viewed from above.

8. The transfer instrument according to claim 2, further comprising
an outer cylinder through which the first shaft is inserted, wherein
the support portion is flexible and formed in a sheet shape,
the support portion is formed to be wider than an inner diameter of the outer cylinder,
the support portion and the medical sheet are retracted into the outer cylinder with the support portion and the medical sheet undergoing curved deformation when the first shaft and the second shaft are moved in a proximal end direction relative to the outer cylinder, and
the support portion and the medical sheet retracted in the outer cylinder are unfolded when the first shaft and the second shaft are moved in the distal end direction relative to the outer cylinder to be exposed from the outer cylinder.

9. The transfer instrument according to claim 8, wherein
a maximum width of the pressing portion is smaller than a width of the support portion and larger than an outer diameter of the second shaft.

10. The transfer instrument according to claim 8, wherein
the pressing portion is flexible, and
a width of the pressing surface is larger than the inner diameter of the outer cylinder.

11. The transfer instrument according to claim 8, wherein
the support portion includes a pair of protrusions protruding upward from both sides of the support surface in a width direction.

12. The transfer instrument according to claim 11, wherein
the pressing portion is provided with a retaining slit into which the pair of protrusions are inserted in a retracted state where the support portion and the medical sheet are retracted in the outer cylinder.

13. The transfer instrument according to any one of claims 1 to 12, further comprising
an endoscope that captures an image of the support portion, the pressing portion, and the medical sheet.
